# EUROPEAN PATENT SPECIFICATION

(11) **EP 0 764 273 B1**
(45) Date of publication and mention of the grant of the patent: **14.10.1998**
(21) Application number: 95920305.0
(22) Date of filing: 08.06.1995
(51) Int. Cl.: G01N 33/68, A61K 38/17, A61K 39/395, A61K 35/14

(54) **ALPHA B CRYSTALLIN FOR USE IN DIAGNOSIS AND THERAPY OF AUTO-IMMUNE DISEASES IN PARTICULAR MULTIPLE SCLEROSIS**
ALPHA B CRYSTALLIN ZUR VERWENDUNG IN DIAGNOSE UND THERAPIE VON AUTOIMMUNKRANKHEITEN, BESONDERS MULTIPLER SKEROSE
CRISTALLINE ALPHA B UTILISEE DANS LE DIAGNOSTIC ET LE TRAITEMENT DE MALADIES AUTO-IMMUNES ET EN PARTICULIER LA SCLEROSE EN PLAQUE

(30) Priority: 09.06.1994 EP 94201653
(43) Date of publication of application: 26.03.1997
(73) Proprietor: NEDERLANDSE ORGANISATIE VOOR TOEGEPAST-NATUURWETENSCHAPPELIJK ONDERZOEK TNO, 2595 CL Den Haag (NL)
(72) Inventor: VAN NOORT, Johannes, M., NL-2288 GJ Rijswijk (NL); VAN SECHEL, Arianne, C., NL-2288 GJ Rijswijk (NL); OUAGMIRI, Mustapha, El, NL-2288 GJ Rijswijk (NL)
(74) Representative: Smulders, Theodorus A.H.J., Ir.
(86) International application number: NL9500203
(87) International publication number: WO9533997

(56) References cited:
- AMERICAN JOURNAL OF PATHOLOGY, vol. 140, no. 2, February 1992 HAGERSTOWN MD, USA, pages 345-356, T. IWAKA ET AL. 'Accumulation of alphaB-crystallin in central nervous system glia and neurons in pathologic conditions.' cited in the application
- CELL, vol. 57, no. 1, 7 April 1989 CAMBRIDGE MA, USA, pages 71-78, T. IWAKI ET AL. 'alphaB-crystallin is expressed in non-lenticular tissues and accumulates in Alexander's disease brain.'
- AMERICAN JOURNAL OF PATHOLOGY, vol. 143, no. 2, August 1993 HAGERSTOWN MD, USA, pages 487-495, T. IWAKI ET AL. 'alphaB-crystallin and 27-kd heat shock protein are regulated by stress conditions in the central nervous system and accumulate in Rosenthal fibers.' cited in the application
- THE EMBO JOURNAL, vol. 13, no. 4, 15 February 1994 OXFORD, GB, pages 945-953, I. NICHOLL ET AL. 'Chaperone activity of alpha-crystallins modulates intermediate filament assembly.' cited in the application
- THE JOURNAL OF BIOLOGICAL CHEMISTRY, vol. 267, no. 11, 15 April 1992 BALTIMORE MD, USA, pages 7718-7725, K. KATO ET AL. 'Copurification of small heat shock protein with alphaB crystallin from human skeletal muscle.'
- THE JOURNAL OF IMMUNOLOGY, vol. 149, no. 4, 15 August 1992 BALTIMORE MD, USA, pages 1444-1451, R. SOBEL ET AL. 'The immunopathology of acute experimental allergic encephalomyelitis induced with myelin proteolipid protein. T cell receptors in inflammatory lesions.'

### SEQUENCE LISTING

### (1) GENERAL INFORMATION:

(i) APPLICANT:
   (A) NAME: Nederlandse Organisatie voor Toegepast Natuurwetenschappelijk Onderzoek TNO
   (B) STREET: Juliana van Stolberglaan 148
   (C) CITY: The Hague
   (D) STATE: Zuid-Holland
   (E) COUNTRY: the Netherlands
   (F) POSTAL CODE (ZIP): 2595 CL

   (A) NAME: van Noort, Johannes Maria
   (B) STREET: c/o Lange Kleiweg 139
   (C) CITY: Rijswijk
   (D) STATE: Zuid-Holland
   (E) COUNTRY: the Netherlands
   (F) POSTAL CODE (ZIP): 2288 GJ

   (A) NAME: van Sechel, Arianne Christine
   (B) STREET: c/o Lange Kleiweg 139
   (C) CITY: Rijswijk
   (D) STATE: Zuid-Holland
   (E) COUNTRY: the Netherlands
   (F) POSTAL CODE (ZIP): 2288 GJ

   (A) NAME: Oagmiri, Mustapha El
   (B) STREET: c/o Lange Kleiweg 139
   (C) CITY: Rijswijk
   (D) STATE: Zuid-Holland
   (E) COUNTRY: the Netherlands
   (F) POSTAL CODE (ZIP): 2288 GJ
(ii) TITLE OF INVENTION: Alpha B crystallin for use in diagnosis and therapy of auto-immune diseases in particular multiple sclerosis.
(iii) NUMBER OF SEQUENCES: 4
(iv) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: PatentIn Release #1.0, Version #1.30 (EPO)
(v) CURRENT APPLICATION DATA:
   APPLICATION NUMBER: PCT/NL95/00203

### (2) INFORMATION FOR SEQ ID NO: 1:

(i) SEQUENCE CHARACTERISTICS:
   (A) LENGTH: 175 amino acids
   (B) TYPE: amino acid
   (C) STRANDEDNESS: unknown
   (D) TOPOLOGY: unknown
(ii) MOLECULE TYPE: protein
(iii) HYPOTHETICAL: NO
(xi) SEQUENCE DESCRIPTION: SEQ ID NO: 1:
   Met Asp Ile Ala Ile His His Pro Trp Ile Arg Arg Pro Phe Phe Pro
   1               5                   10                  15
   Phe His Ser Pro Ser Arg Leu Phe Asp Gln Phe Phe Gly Glu His Leu
               20                  25                  30
   Leu Glu Ser Asp Leu Phe Pro Thr Ser Thr Ser Leu Ser Pro Phe Tyr
           35                  40                  45
   Leu Arg Pro Pro Ser Phe Leu Arg Ala Pro Ser Trp Phe Asp Thr Gly
       50                  55                  60
   Leu Ser Glu Met Arg Leu Glu Lys Asp Arg Phe Ser Val Asn Leu Asn
   65                  70                  75                  80
   Val Lys His Phe Ser Pro Glu Glu Leu Lys Val Lys Val Leu Gly Asp
                   85                  90                  95
   Val Ile Glu Val His Gly Lys His Glu Glu Arg Gln Asp Glu His Gly
               100                 105                 110
   Phe Ile Ser Arg Glu Phe His Arg Lys Tyr Arg Ile Pro Ala Asp Val
           115                 120                 125
   Asp Pro Leu Ala Ile Thr Ser Ser Leu Ser Ser Asp Gly Val Leu Thr
       130                 135                 140
   Val Asn Gly Pro Arg Lys Gln Val Ser Gly Pro Glu Arg Thr Ile Pro
   145                 150                 155                 160
   Ile Thr Arg Glu Glu Lys Pro Ala Val Thr Ala Ala Pro Lys Lys
                   165                 170                 175

### (2) INFORMATION FOR SEQ ID NO: 2:

(i) SEQUENCE CHARACTERISTICS:
   (A) LENGTH: 8 amino acids
   (B) TYPE: amino acid
   (C) STRANDEDNESS: unknown
   (D) TOPOLOGY: unknown
(ii) MOLECULE TYPE: protein
(iii) HYPOTHETICAL: NO
(xi) SEQUENCE DESCRIPTION: SEQ ID NO: 2:
   Ile Pro Ala Asp Val Asp Pro Leu
   1               5

### (2) INFORMATION FOR SEQ ID NO: 3:

(i) SEQUENCE CHARACTERISTICS:
   (A) LENGTH: 4 amino acids
   (B) TYPE: amino acid
   (C) STRANDEDNESS: unknown
   (D) TOPOLOGY: unknown
(ii) MOLECULE TYPE: protein
(iii) HYPOTHETICAL: NO
(xi) SEQUENCE DESCRIPTION: SEQ ID NO: 3:
   Tyr Leu Arg Pro
   1

### (2) INFORMATION FOR SEQ ID NO: 4:

(i) SEQUENCE CHARACTERISTICS:
   (A) LENGTH: 13 amino acids
   (B) TYPE: amino acid
   (C) STRANDEDNESS: unknown
   (D) TOPOLOGY: unknown
(ii) MOLECULE TYPE: protein
(iii) HYPOTHETICAL: NO
(xi) SEQUENCE DESCRIPTION: SEQ ID NO: 4:
   Ala Pro Ser Trp Phe Asp Thr Gly Leu Ser Glu Met Arg
   1               5                   10

## Description

The invention relates to the field of immunology, in particular to the field of auto-immune diseases. More in particular, it relates to the diagnosis and therapy of auto-immune diseases, especially multiple sclerosis. Auto-immune diseases are diseases wherein the defense mechanism of the body against unwanted materials, such as bacteria, viruses, etc. looses the ability to discriminate between unwanted and self material and attacks parts of the own material, resulting in destruction of essential tissues and functions. To allow for a better understanding of the present invention a short introduction to how this defense mechanism operates is given below.

### The immune system and its control

The immune system of man and animal is a delicately balanced system designed to monitor and remove pathogens and foreign substances from the living body. Every day, each individual encounters numerous different bacteria, viruses or other substances that may jeopardize normal body functions when residing or even propagating within body tissues. Immunologists refer to any proteinaceous material foreign to the body itself as 'antigen'. The immune system comprises a variety of different cells that may respond to antigens from every conceivable source.

Specific responses of the immune system are controlled by a particular type of cells, called helper T cells, that act as monitoring and regulatory cells. Such helper T cells may activate cytotoxic T cells (to destroy affected cells or tissues), B cells (to produce and secrete antibodies) or macrophages (to ingest material and secrete protein-degrading enzymes and damaging oxidative radicals). Thus, helper T cells play a pivotal role in determining when the immune system comes into action and against exactly which structures it will be targeted.

This latter element, the target specificity of the immune response as recognized by helper T cells, is defined by a structural sample of the antigen, viz. protein fragments derived from it. As a rule, helper T cells screen for the presence of antigen by examining sets of protein fragments that are presented to them by so-called accessory cells, frequently macrophages. Such accessory cells ingest antigen and partially digest the material by protein-degrading enzymes, irrespective of the nature or source of the antigen. Next, resulting protein fragments derived from the antigen are trapped (still within the accessory cell) by special proteins designed to capture and present protein fragments. These proteins, encoded in the major histocompatibility complex (MHC) class II region in the genome, are referred to as class II MHC proteins. MHC proteins, complexed in a 1:1 ratio with a protein fragment emerging from the degradative processes within the accessory cells, become surface exposed and can reside at the cell surface for days. Thus, they will present to passing helper T cells a structural sample of antigens in the form of the captured protein fragments [1].

The immune system comprises a population of helper T cells, each of which possesses a surface receptor structure with its own individual target specificity [2]. Together, however, the many millions of different helper T cells receptors on the different cells are capable of recognizing virtually every protein fragment. This is a prerequisite for the immune system's potential to recognize an endless variety of structurally different entities. At the same time however, it poses a significant risk that responses may occur also to structures that belong to the body itself. An accurate balance between responding to possibly pathogenic antigen and being tolerant to the body's own tissues is crucial to a healthy immune system [3].

Unfortunately, the balance between aggression and tolerance is not always maintained. Especially tissue-specific structures within the body may become the target of autoreactive immune responses and disease may ensue [4]. In case of aberrant responses to insulin-producing islets of Langerhans within the pancreas, insulin-dependent diabetes may result. When cartilage structures are attacked, rheumatoid arthritis follows and if something within the brain's white matter triggers the immune system, multiple sclerosis may develop. Although these diseases result in quite different symptoms in the individuals they affect, immunologists consider them to have similar origins: an uncontrolled and perpetuating immune response against one or more antigens within the body's own tissues: autoimmunity.

In the western world, about 3 % of all people suffer from autoimmune responses. About one in every thousand suffers from multiple sclerosis (MS). MS is characterized by scattered regions of inflammation (lesions) within white matter of the central nervous system, brain and spinal cord [5]. This white matter is mainly composed of a material specific to central nervous system (CNS) tissue, viz. myelin, that is wrapped around axons in layers of insulating membranes. Nerve signals controlling every imaginable body function travel through axons from one nerve cell to another. Unlike current through electric wire, however, signal conduction in the CNS is achieved by so called saltatory conduction. Electrochemical potentials are not gradually transported along the entire axon length, but jump between small spots of naked axons (nodes of Ranvier) that exist between the patches of myelin.

By jumping from spot to spot, nerve signal transduction in the central nervous system is rapid and efficient. In MS, focal inflammatory responses in the CNS cause the degradation of the myelin layer and their complete removal from axons. Demyelinated axons become much less efficient in nerve signal conduction since the saltatory mode of transduction is no longer possible. Nerve signals will thus take more time to travel the required distances and they will loose part of their potential along the way. Even if the inflammatory response passes, damage persists since remyelination of naked axons occurs to an only very limited extent. Instead, scar tissue develops. It is for this reason that damage in the CNS as the result of MS slowly accumulates.

It is generally believed that the target antigen for the autoimmune response in MS is to be found in CNS myelin [5, 6]. Myelin consists of differentiated oligodendrocyte plasmamembranes, containing about 75% lipids and about 25% proteins [7]. Since helper T cells only respond to protein fragments, no major role is anticipated for the different lipid components of myelin in triggering autoimmune responses. The 25% protein mass of myelin however, is highly complex in composition. Several levels of complexity may be discerned.

First, CNS myelin contains a large number of different proteins. Predominant protein components exist, viz. myelin basic protein (MBP) and proteolipid protein (PLP), that represent about 15 and 35% of the total protein mass, respectively. In addition, however, a multitude of other protein components have been found to be specifically associated with CNS myelin. Some of these proteins have been under study as the result of the availability of monoclonal antibodies. These are for example myelin-associated glycoprotein (MAG) and myelin/oligodendrocyte glycoprotein (MOG), which represent only very minor amounts of protein mass in the order of less than 1 %. Numerous other proteins including a variety of different enzymes have been found in myelin as well [reviewed in 8].

Secondly, molecular biological studies of myelin proteins have consistently revealed the presence of different isoforms [reviewed in 9]. More as a rule rather than as an exception, primary transcripts of myelin proteins may be spliced differentially into different isoforms. Such molecular species differ from one another in the number of exons they represent. For example, MBP is currently known to exist in at least six different isoforms in mice.

Thirdly, every myelin protein examined to date contains at least one type of post-translational modification. These modifications include acetylations, acylation, glycosylation, methylation, phosphorylation etc. Finally, both isoform expression and extent and patterns of post-translational modifications may vary in time according to developmental stage or health. In the development of mice, constantly different ratios of different MBP isoforms can be found in the brain. When the animals are struck by infections in the CNS, such patterns may change and different modifications may appear. Without any additional information, no single myelin protein may be considered to be a more relevant antigenic target than any other myelin protein. The mere abundance of e.g. MBP or PLP does not immediately imply these proteins to be any more immunogenic than other myelin components. Numerous examples exist indicating that helper T-cell responses to protein mixtures may well be directed at very minor components [8]. Despite this consideration, much of the effort in immunological research on MS has been devoted to MBP. Without any doubt, the simple fact that MBP is a water soluble and readily available protein for experimental studies has played a major role in its selection as candidate autoantigen.

However, so far there is no evidence that any of the above mentioned proteins represents a major auto-antigen in auto-immune diseases, particularly MS.
The need for identifying such an auto-antigen is apparent from the above, if only to be able to diagnose MS at a much earlier stage and to design rational intervention strategies. Moreover, knowledge of the auto-antigen can be used to develop specific therapies for MS instead of the aspecific immunemodulation which is used to date. Based on the auto-antigen it will be possible to develop vaccines or schemes for inducing tolerance [10-12]. The present invention provides an important candidate for being a major auto-antigen in the development of MS and a target for diagnosis and therapeutic intervention.

### A highly immunodominant CNS myelin protein: alpha B crystallin

By applying a novel approach to the study of myelin-reactive human helper T cells, a new antigen has now been identified that is likely to serve as crucial autoantigen in inflammatory responses in MS.

In previous studies documented throughout the years, the approach has always been to purify an individual protein from CNS myelin and subsequently examine its ability to trigger helper T cell responses in man or demyelinating T-cell responses in laboratory animals. Although relevant information on the principles of autoimmune responses could thus be generated, this approach did never address the crucial question whether or not the selected antigen was actually immunodominant to helper T cells in the context of all myelin proteins together. Yet, it is very likely that a challenge of the immune system with all myelin proteins at the same time is close to what happens in MS, at least in ongoing disease.

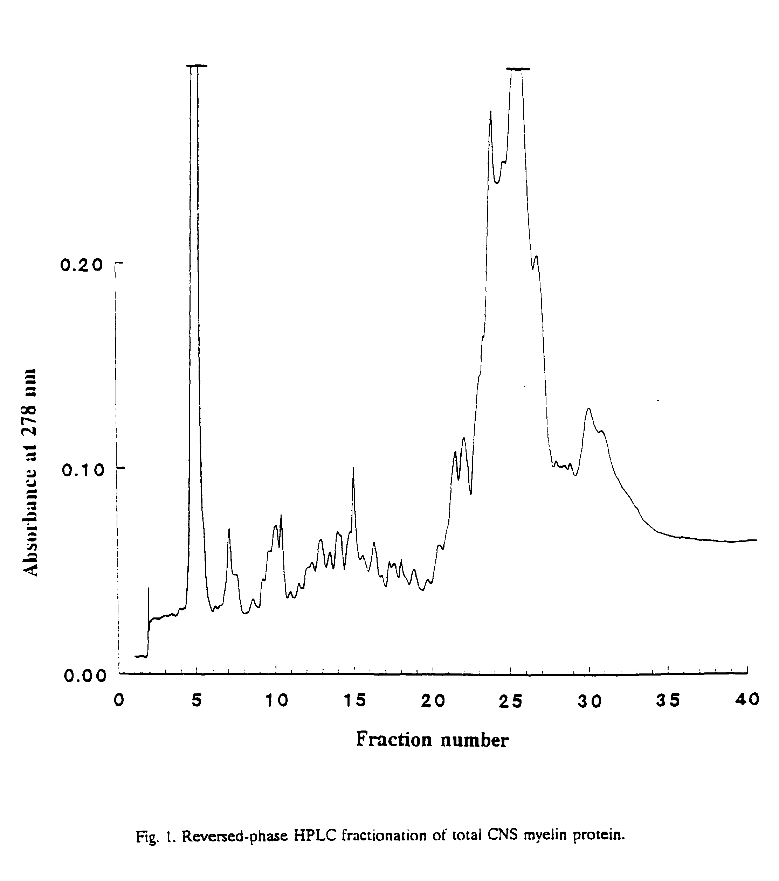

Although the notion that a comprehensive comparison of all respective immunogenicities of myelin proteins has long been warranted, its technical implementation has always posed serious problems. A major problem has been the fact that most of the myelin proteins, being membrane proteins, are very hydrophobic and poorly soluble in aqueous solution. Yet, solubility in aqueous environment is a strict prerequisite for performing immunological assays on these proteins. We have recently circumvented the problems associated with poor solubility by combining exhaustive protein delipidation with a quantitative two-step transfer of all CNS myelin proteins from organic solvents to aqueous solutions without losing their solubility [13]. As a result, we have been able to make all proteins available for immunological studies. Also, delipidated myelin proteins could be separated by reversed phase HPLC with a resolution beyond what had been reported sofar (see Fig. 1). In this way, a comparative study could be performed in which helper T cell responses were assayed in response to separated myelin proteins after priming of the T cells with all CNS myelin proteins in a single preparation.

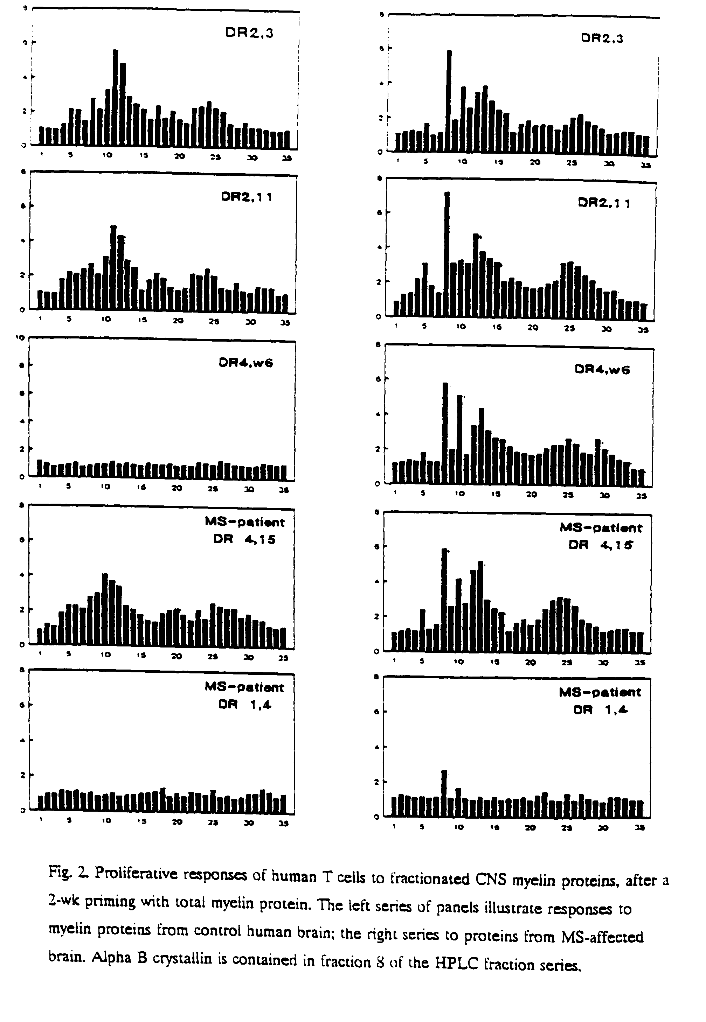

Moreover, the examination was performed in parallel experiments using human myelin proteins from MS-affected brains in one set of tests and myelin preparations from control brains in another. Using these sets of priming/test antigens, peripheral blood T cells from twenty-seven different individuals have been tested for proliferative responses to the fractionated myelin proteins. This novel approach has yielded unexpected and very useful data. They are represented in Fig. 2 (given on a separate sheet).

First, helper T cells from all donors were found responsive mainly to minor myelin proteins whereas only very weak responses were detected to either of the two predominant components MBP or PLP. This low response to the major protein components was found with T cells derived from both healthy and MS-affected donors. Secondly and most importantly, a surprising difference was observed in responses against myelin proteins isolated from MS-affected brain. Only when using this set of proteins as test antigens, a predominant proliferative T cell response turned out to be directed at a single HPLC protein fraction in all cases examined. T cell samples from healthy donors and MS-affected ones consistently showed the highest response to this particular protein fraction.

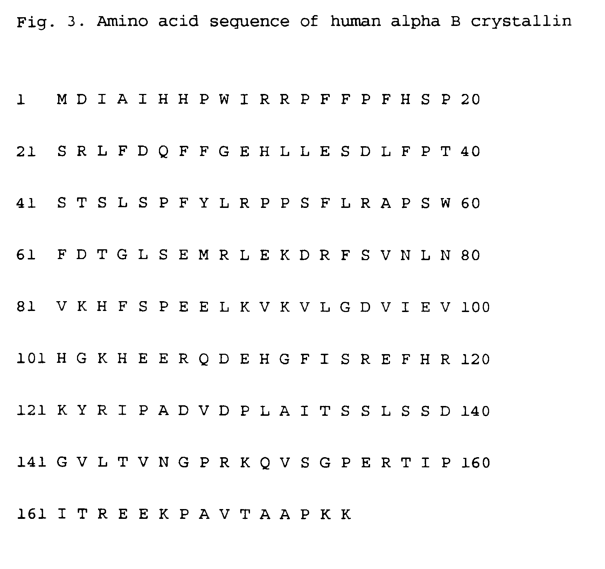

Close examination of this protein fraction revealed that it almost exclusively contained a 23 kDa protein which, upon sequencing, was identified as human alpha B crystallin. Although direct sequencing of the purified protein was impossible due to an N-terminal modification, six tryptic peptides derived from it consistently yielded sequences identical to alpha B crystallin (Table 1.). The protein contains 175 amino acids; only four amino acids are different between the human and the bovine form of the protein. The amino acid sequence of human alpha B crystallin is given in Fig. 3.

### Alpha B crystallin as autoantigen in MS

Until 1989, crystallins including alpha B crystallins were believed to be proteins exclusively located in the eye lens. Upon screening different tissues with antisera against alpha B crystallin, however, its presence was also established in striated (skeletal) muscle, kidney and CNS tissue 14, Kato et al., J. Biol. Chem. 267(11), 1992, 7718-7725. Within the normal CNS, alpha B crystallin is mainly found in oligodendrocytes located in the white matter. Interestingly, previous studies on alpha B crystallin have revealed that its sequence is highly homologous to that of the family of small heat-shock proteins (or stress proteins) [15]. Consistent with its role as heat shock protein,alpha B crystallin has been found to exhibit chaperone characteristics [16].

In addition, immunohistochemical studies have confirmed that cellular levels of alpha B crystallin increase as a result of stress. In a number of pathological conditions including e.g. Alzheimer's disease and Alexander's disease levels of alpha B crystallin in the CNS have been found to be elevated 17, 18, Iwaki et al., Cell 57(1), 1989, 71-78. In the latter condition, significant amounts of alpha B crystallin are found to be accumulated inside astrocytes in the form of fibres, so-called Rosenthal fibres. Our own preliminary data obtained by immunohistochemical staining of CNS tissue with polyclonal anti-alpha B crystallin antibodies indicate enhanced expression within or closeby MS lesions as compared to unaffected regions of white matter. Thus, not only sequence homologies but also actual tissue expression of alpha B crystallin point to a role of this protein as heat-shock protein.

The combination of immunodominant target antigen among all different myelin proteins and its role as heat shock protein within glial cells renders alpha B crystallin a highly attractive candidate autoantigen. Our observation that only when using MS-affected myelin preparations a clear T cell response is found to the protein may well be explained by increased levels of the protein in such preparations relative to control preparations. However, particular disease-associated modifications of the protein may still be considered relevant as well. It is known that alpha B crystallin may be the target of various modifications including e.g. phosphorylations. If alpha B crystallin is the autoantigen in MS, its role as heat shock protein may explain why epidemiological studies on MS have pointed to a role of viral infections in the development of MS despite the fact that no single type of virus has been identified which has consistently been found to be associated with MS [19].

Viral infections within the CNS, albeit involving different types of viruses, may readily lead to a common side effect viz. enhanced local expression of alpha B crystallin as the result of stressful inflammatory mediators. At the same time, local infection will result in a local increase of class II MHC molecules e.g. on infiltrating macrophages or on resident astrocytes [20]. It is not difficult to envisage that the combination of enhanced expression of antigen presenting class II MHC molecules and increased levels of immunodominant alpha B crystallin at the same time and in the same location may result in passing a threshold for triggering alpha B crystallin-directed helper T cell responses. Also the perpetuation of such responses is easily explained along the same lines. The immunodominance of alpha B crystallin has been observed in all human HLA-DR backgrounds examined sofar, including the DR2 type that is associated with MS in Western Europe.

### Diagnosis and therapy in MS based on alpha B crystallin as autoantigen.

The new and surprising finding that indeed, a single immunodominant myelin protein exists for all human class II MHC backgrounds and that this protein has both structural and functional characteristics of a heat shock protein as well, opens a series of possibilities for new approaches in diagnostics and therapy in auto-immunity related diseases, especially MS.

Diagnostics in MS has always been a problem. Not only the intrinsic variety of different clinical symptoms that may be caused by local afflictions in the CNS, also the time course of clinical signs considered to be characteristic to MS renders a rapid diagnosis virtually impossible. If relapsing remitting phenomena are considered to be of crucial importance in establishing the diagnosis, one will simply have to monitor disease for an extended period of time. The recent advent of magnetic resonance imaging as a diagnostic tool in monitoring local CNS inflammations has contributed greatly to diagnostics in MS but has not yet completely solved the time problem. A lengthy procedure for the diagnostic confirmation of MS is undesirable, not only for the MS patients themselves but also for the possible efficacy of therapeutic approaches. Specific immune responses to alpha B crystallin may assist in diagnosing MS.

First, the formation of immunoglobulins, specific to alpha B crystallin, may be assessed in tissues and/or body fluids such as blood or cerebrospinal fluid and they may be specifically enhanced in MS. Such enhanced antibody responses may be employed as a diagnostic marker for MS in the design of antigen-based kits to monitor antibody responses.

Antigen based kits should be read to include, but not be limited to ELISA's, RIA's, SPIA's and for DIA's (differing foremost in the kind of label attached to the specific binding reagent for the antibody to alpha B crystallin) in any suitable format such as sandwich-, competition- or agglutination-assays.

A suitable format is a sandwich-assay, wherein alpha B crystallin is provided on a solid phase, whereby a sample suspected of containing the antibodies to alpha B crystallin is contacted with said solid phase, so that it can bind to the antigen, after which the solid phase is contacted with labelled antigen, or with a labelled antibody directed to the alpha B crystallin antibody. Instead of whole antigens and/or antibodies it is of course very well possible to use specifically binding parts thereof.

Many labels are known and can be applied in assays according to the invention. Such labels include enzymes, which usually need a substrate to give a signal, particle sols, such as metal (gold) sols, coloured latex particles, dyestuffs, fluorescent materials and radioactive materials. The assay itself can be in a laboratory format, or for use in the Doctor's Office or even for home use. The man skilled in the art will be able to construct a suitable assay based on the invention.

Alternatively, T cell responses and in particular helper T cell responses to alpha B crystallin may serve as diagnostic marker in MS. A variety of methods may be envisaged for the detection of augmented T cell responses such as screening for activation markers on alpha B crystallin-specific T cells in body fluids including cerebrospinal fluid, examining frequencies of specific T cells or enhanced production of cytokines in response to alpha B crystallin. Thus, increased immune responses against alpha B crystallin, either humoral or cellular, which may be specifically associated with MS may be employed to assist diagnosis.

For the development of antigen-specific therapeutic approaches in MS, a large variety of strategies are already available, and others are yet to be developed [reviewed in 21]. Antigen-specific strategies have been tested in animal models of autoimmunity and several of these methods have been found effective in preventing or ameliorating disease. Such specific therapies may be aimed at intervention in the productive formation of trimolecular interactions between antigen, T-cell receptor and HLA molecule which result in activation of the T cell involved. Alternatively, antigen (or fragments of it) may be administered in such a way that specific tolerance rather than immune responsiveness is triggered. Thus, specific intervention may be achieved at different levels. The administration of antigen via tolerance-inducing routes may be employed to mount tolerance to a specific autoimmunity-inducing antigen. For example, oral administration of target antigen may lead to a state of tolerance in which autoimmunity can no longer be induced. The same may be achieved by frequent administration of either very low or very high doses of antigen over an extended period of time. Either dosage may fail to induce immunity but, instead, may finally result in a state of non-responsiveness or tolerance to the antigen used. Also thymic implantation of antigen may result in specific tolerance since the thymic environment by itself appears to be designed to induce tolerance in T cells that locally respond to their target structure. These routes of administration designed to induce tolerance, or future alternatives for it, may be taken with whole antigen or selected fragments derived from it either as free entities or as part of a more complex structure (e.g. encapsulated or expressed by micro-organisms).
Also, antigen can be administered together with agents that interfere with the co-stimulatory signals that are essential to T cell activation.

It has recently become clear that the recognition of antigen/HLA complexes by the T-cell receptor alone is not sufficient for productive activation of T cells. For this, additional interactions between several accessory molecules on the surface of antigen-presenting cells and complementary molecules on the surface of T cells are required. Recognition of antigen/HLA without these accessory interactions may not only fail to activate the T cell, it may even lead to a state of antigen-specific anergy (unresponsiveness) of the T cell [22]. This phenomenon may be employed to induce specific tolerance in man or animals [23]. For example, administration of antigen/HLA complexes (as free or membrane-bound preparations) may specifically anergize the antigen-specific T cells rather than activate them. Also the administration of antigen on B cells, which lack essential accessory molecules to activate naive (previously unactivated) T cells may have the same effect. Finally, specific antibody preparations (or other therapeutic agents) targeted at accessory molecules, when given in conjunction with antigen could also result in the anergy of T cells specific to the co-administered antigen. It is to be expected that yet other strategies to the induction of antigen-specific anergy will be developed in the future but these will remain crucially dependent on knowledge of the antigen.

Unresponsiveness (or non disease-inducing responsiveness) may not only be achieved by a specific route of antigen administration, but also by administration of selected fragments of the antigen. In animal models of autoimmunity, it has been demonstrated that autoimmunity-inducing antigens may contain sequences which may prevent or ameliorate the development of autoimmune disease when given as separate entities from the intact antigen. Thus, treatment with selected fragments of antigen may have the opposite effect as treatment with whole antigen. An interesting variant to this concept is the administration of slightly altered sequences of the antigen. Antigen fragments that may normally be involved in the activation of autoreactive T cells may be altered somewhat in sequence in order to achieve the opposite effect: specific unresponsiveness of antigen-specific T cells. Such slightly altered antigen fragments (APL's or 'altered peptide ligands') may act as antagonists or modulator peptides. These may specifically engage TCR with an authentic specificity to the autoantigen in such a way that productive accessory interactions, required for T cell activation, are prevented. The effect is similar to that in the case of preventing accessory signals by other methods: specific unresponsiveness of the T cell involved. Although it cannot be predicted beforehand which alterations in the antigen sequence may or may not produce such an 'antagonistic/modulator' effect, it seems likely that these effects are the strongest with sequences very similar to the authentic antigen sequence.

Another target for antigen-specific intervention in T cell mediated autoimmunity is the T cell itself. Just like the structure of antigen is unique to its identity, the structure of the T-cell receptor (TCR) is unique to the recognition of its antigen target. Thus, TCR structures may be identified that are specific to the recognition of a given antigen. These structures can be found in the variable-, joining- or diversity-coding regions of the TCR gene for either alpha or beta chain. Structures specific to the recognition of a given antigen may be designated clonotypic or idiotypic TCR structures. Again, experiences in animal models for autoimmunity have demonstrated possibilities for intervention in disease by specifically aiming intervention at these clonotypic/idiotypic TCR structures. First, this may be achieved by vaccination with antigenspecific T cells themselves. T cells to any antigen may be selected by culturing in vitro and vaccination may be carried out with inactivated populations of such presumed autoreactive T cells.

Vaccination is aimed at raising an immune response (either cellular or humoral) in the diseased individual specific to the antigen-specific T cells themselves in order to achieve their elimination from the body. Secondly, the same may be achieved by vaccination strategies employing not the complete T cell as therapeutic agent, but only relevant structures such as the TCR alone or even just small sequences corresponding to clonotypic or idiotypic determinants in the TCR. For such strategies, TCR or sequences derived from it may also be used as part of larger structures. Although not yet demonstrated, antigen-specific (idiotypic) features of T cells may not only be reflected in structural elements of the TCR alone but could in principle also be reflected in alternative specific structures either within or on the surface of cells.

Development of antigen-specific therapies in autoimmunity has been an elusive goal for many years. Advances in experimental animal models where trigger autoantigens are known by preselection have shown an increasing number of successful approaches to prevent even ongoing autoimmunity [21]. Either the antigen structure itself or features of the specific T cells activated by it may be employed for the rational design of therapy. Also in human autoimmunity, identification of all idiotypic and other relevant features of autoreactive T cells that could be involved in autoimmunity is fully dependent on identification of the relevant antigen. In other words: the identification of the antigen is the key to selection and characterization of the responding T cells.

Also the assessment of genetic predisposition to MS may be assisted by an analysis of alpha B crystallin gene sequences in the human genome. The possible occurrence of polymorphisms within the alpha B crystallin gene or adjacent sequences on human chromosome 11 may provide clues to altered (enhanced) expression or modified protein structures, both of which could be relevant to the capacity of the gene product to trigger autoimmune responses and thus, to the likelihood of developing MS.

Alpha B crystallin does not only provide a structural trigger/target for autoimmunity in MS, limited to white matter in the CNS, it may also provide an explanation of how tissue-specific autoimmunity against CNS myelin may have a multifactorial onset [19]. Behaving as heat-shock or stress protein, alpha B crystallin may locally be expressed in high levels in response to a variety of challenges such as persistent neurotropic infections, ischaemic attacks or physical injury. Together with its immunodominance to human T cells, increased levels of alpha B crystallin may occasionally be expected to raise beyond the threshold for T cell activation, especially if e.g. infections at the same site raise local class II MHC expression of microglia and astrocytes. This notion may provide the basis for yet another approach to therapy, i.e. aimed at reducing alpha B crystallin levels in the CNS. Molecular biological approaches involving for example antisense DNA or RNA may be developed in order to specifically block or reduce expression of alpha B crystallin in the target tissue. Such goals may be achieved e.g. by gene therapy or by infection with altered viruses that produce antisense nucleic acids or so-called "hammerhead" ribozymes directed at destroying the alpha B crystallin messenger or reducing its levels. It may be envisaged that in the future also other strategies may be developed along this line that are based upon the identification of alpha B crystallin as target autoantigen.

Thus the invention provides an important major auto-antigen (alpha B crystallin) for use in the diagnosis or therapy of auto-immunity related diseases, in particular Multiple Sclerosis. It will be clear from the afore going that alpha B crystallin can be used in many ways according to the invention. It is, however, also true that it may not be necessary to use complete alpha B crystallin.

In many instances it will also be possible to apply only part of the whole protein or derivatives of the protein and the like, which have similar activity as alpha B crystallin. Accordingly these parts and/or derivatives are also part of the present invention.

For diagnosis it has been explained hereinabove how assays can be carried out and how test kits can be assembled on the basis of the alpha B crystallin.

For therapeutic purposes, for instance when the antigen is to be administered in a composition for induction of tolerance it will be clear that depending on the route of administration (parenteral, enteral) and the patient the dosages may vary. Generally they will lie between 1 ng/kg body weight/day and 1 mg/kg body weight per day or other efficacious doses.

Such compositions may of course comprise suitable excipients.
How the alpha B crystallin is obtained is not important. It may be isolated from myelin material or other tissues or it may be produced recombinantly through suitable host cells. Fragments (peptides) may be produced synthetically.

For recombinant production it is necessary to provide the gene coding for alpha B crystallin, which can be easily done because the amino acid sequence is known.
For instance techniques like PCR will be very helpful in this respect. Having the gene provides yet other useful diagnostic and therapeutic tools according to the invention.

It will be clear that PCR and NASBA and the like can be used to diagnose enhanced expression of alpha B crystallin. The usefulness of antisense approaches has already been mentioned before. For these approaches it will also be clear that it is not always required to use the entire gene or an unaltered gene. Fragments and/or derivatives may also be used.

For expression of the gene many cells, vectors and expression regulators such as promoters, enhancers and the like are known. If post-translational modifications are necessary or if a signal sequence needs to be removed, it may be best to employ eukaryotic cells such as CHO cells and the like.

It will be clear for the man skilled in the art that it is also possible to obtain derivatives of alpha B crystallin, which will work as antagonists and thus block the T cell response to alpha B crystallin. Another way of arriving at such antagonists is by providing anti-idiotype antibodies, which have a complementarity determining region which is almost an internal image of the epitope of alpha B crystallin recognized by the T cell receptor. It is also possible to identify the responsible T cell receptor and prepare antibodies thereto.

These and any other antibodies may be prepared according to any of the well known techniques. Preferably monoclonal antibodies are prepared. It is usual practice to produce antibodies in rodents, however, if they have to be applied in humans, these will usually lead to immuneresponse.

Techniques to reduce the immunogenicity of these antibodies by providing fragments or hybrid human/rodent antibodies, as well as techniques to engineer these antibodies into humanized antibodies (CDR-grafting) are all within the art.

As stated herein before it is also possible to prepare vaccines based on the responsible auto-reactive T cells. For this purpose the T cells have to be inactivated.

Instead of complete T cells their receptors (TCR's) or parts of derivatives can be used. They can, like the antigens and the antibodies, be obtained in a variety of ways, including isolation, synthesis, genetic engineering, etc.

Any and all vaccines can comprise the usual excipients. The invention will now be explained in greater detail in the following examples.

### EXPERIMENTAL SECTION

### 1. Materials and Methods

### 1.1 Preparation of myelin proteins for immune assays

White matter samples from human brains obtained by rapid autopsies were kept frozen at -80°C until use. Samples including white matter lesions were obtained from 13 definite MS patients and another set of samples from 21 control subjects. CNS myelin was purified from white matter samples as described by Norton and Poduslo [24]. Purified myelin preparations from either MS patients or control subjects were pooled separately and stored lyophilized at -20°C until use.

For the preparation of whole myelin protein as stimulatory antigen, purified myelin was delipidated according to van Noort et al. [13]. Briefly, myelin was dissolved in 80% (v/v) tetrahydrofuran, 20% (v/v) water, 0.1% (v/v) trifluoroacetic acid and passed over Sephadex LH-60 in order to separate proteins from (glyco)lipids. Protein-containing eluate was pooled and the proteins were precipitated from the solution by adding diethylether. Precipiated proteins were spun down by centrifugation and lyophilized. Delipidated myelin proteins were subsequently dissolved in 2-chloroethanol containing 0.1% (v/v) trifluoroacetic acid at a concentration of approximately 4 mg/ml and dialyzed against four changes of water in regenerated cellulose acetate dialysis membranes. The final protein concentration in the fully aqueous solution was determined by amino acid analysis. The aqueous solutions of total myelin proteins were kept at +4°C since any freezing of such preparations leads to irreversible precipitation of the more hydrophobic protein molecules.

Fractionation of all myelin proteins for use as test antigen in the assays was performed as described previously by van Noort et al [13; see Fig. 1] using a reversed-phase column with a C3 matrix (Beckman Instruments, San Ramon, CA, USA). Following collection and lyophilization of 40 protein fractions as obtained by HPLC, the contents of each fraction were dissolved in 2-chloroethanol, 0.1 % trifluoroactetic acid and dialyzed against water as described above. All aqueous protein fractions were kept at +4°C until use. Based upon amino acid composition analyses of protein samples, equal amounts of total protein mass were taken from both MS-affected and control myelin samples for HPLC fractionation and testing in the proliferation assays.

### 1.2. Testing of bulk T-cell responses to myelin proteins.

Peripheral blood mononuclear cells (PBMC) were obtained by lymphophoresis from twenty-seven HLA-typed donors amongst which five patients with definite MS according to the Poser criteria. PBMC were isolated according to routine procedures and stored at -196°C until use.

PBMC from each donor were grown at 37°C in a humidified stove under a 5% CO₂-atmosphere in 96-well round-bottom microtiter plates. PBMC were seeded at 2.10⁵ cells per 200 µl in RPMI1640 culture medium (Dutch modification) supplemented with 10 mM HEPES, 2 mM glutamine, 1 mM sodium pyruvate, 0.1 mg/ml streptomycin, 100 U/ml penicillin and 10% (v/v) pooled human serum in the presence of total myelin proteins at a final concentration of 25 µg/ml. After seven days, 100 µl of supernatant medium was removed and growing T cells were restimulated by adding 100 µl of fresh RPMI1640 culture medium containg 1.10⁵ irradiated (30 Gy) autologous PBMC and fresh total myelin protein at a concentration of 50 µg/ml. After eleven days, human recombinant IL-2 was added to a final concentration of 50 U/ml. After fourteen days, cells were collected and tested in triplicate for proliferation against HPLC-fractionated proteins in a standard proliferation assay.

A fixed sample of the contents of each HPLC fraction was added to wells containing 5.10⁴ cultured T cells and 5.10⁴ irradiated autologous PBMC in 200 µl culture medium. After three days, 0.6 µCi [³H]-thymidine was added to each well and following another 18 h, cells were harvested and thymidine incorporation was determined using a betaplate counter. By using the above approach, the protein concentration in each well during the proliferation assay varied according to the protein contents of the corresponding HPLC fraction. For those fractions containing either MBP or PLP, the predominant proteins of myelin, final protein concentration in the well was calculated to be about 50 µg/ml on the basis of amino acid analysis of a sample from the original HPLC fraction. Total protein concentrations of HPLC fractions that contain minor proteins were about five- to ten-fold less. The effects of protein concentration upon the qualitative aspects of the result were examined by testing proliferative responses to dilutions of HPLC fractionated proteins. The results obtained were consistent with the dilutions used and revealed no anomalies (data not shown).

### 1.3 Purification and identification of alpha B crystallin

The results obtained in the proliferation assays revealed consistent and strong proliferative responses to one particular protein fraction derived from MS-affected myelin (see below under "Results"). A sample of this fraction, designated by sequential numbering HPLC fraction 8, was analyzed by SDS-polyacrylamide gelelectrophoresis as well as by additional reversed-phase HPLC. Re-chromatography by HPLC was performed by using another type of reversed phase HPLC column, viz. a mixed C1/C8 matrix (Pharmacia LKB, Bromma, Sweden) instead of a C3 matrix (Beckman Instruments, San Ramon, CA, USA) and another eluent, viz. acetonitrile instead of a tetrahydrofuran/acetonitrile mixture.

As described under "Results", both SDS-PAGE and RP-HPLC analysis of the contents of the highly immunogenic fraction 8 revealed the almost exclusive presence of a protein with an apparent mass of approximately 23 kDa. Using a novel preparation of MS-affected myelin as a source, this 23 kDa protein was purified to apparent homogeneity by sequential RP-HPLC using a C3 matrix and tetrahydrofuran/acetonitrile as an eluent in the first step and a mixed C1/C8 matrix and acetonitrile as an eluent in the second and third steps. Step three differed from step two in the gradient applied; 0.1% (v/v) trifluoroacteic acid was used as a solvent throughout.

The purified 23 kDa protein was examined by SDS-PAGE and analyzed by amino acid composition analysis. The protein was also subjected to amino acid sequencing using an Applied Biosystems 470A Model on-line equipped with an Applied Biosystems Model 120A PTH amino acid analyzer. In order to generate fragments of the 23 kDa protein for sequencing, 100 µg of purified 23 kDa protein was dissolved in 100 µl 50 mM Tris-HCl pH 7,8 and supplied with 1 µg bovine spleen trypsin. After 24 h at 37°C, the tryptic digest was fractionated by RP-HPLC on a C18 matrix column using a gradient of acetonitrile in 0.1% (v/v) trifluoroacetic acid. Individual fragments were purified by additional RP-HPLC using 50 mM ammoniumacetate pH 5.8 as a solvent and acetonitrile as an eluent.

For the generation of a polyclonal antiserum against the immunogenic 23 kDa protein, a rabbit was immunized twice with 20 µg of purified 23 kDa protein emulsified in complete Freund adjuvant (CFA) and a third time with 100 µg of protein in CFA at six-week intervals. By using western blotting according to standard procedures, the antibodies raised were tested on recognition of SDS-PAGE separated contents of the original HPLC fraction 8, of the corresponding fraction derived from control myelin, on purified 23 kDa protein used for the immunization, on purified MBP and, finally, on purified alpha B crystallin and alpha A crystallin from bovine eye lens. Both A and B chain of alpha crystallin from bovine lens were purified from a commercial preparation of alpha crystallin (Sigma Chemical Co, St. Louis, MO, USA) by reversed phase HPLC. Prior to application onto a reversed phase column, alpha crystallin was dissolved in 8 M urea, 10 % acetic acid and kept at 37_C for 1 h in order to completely dissociate A and B chains. Using a mixed C1/C8 reversed phase matrix, application of a gradient of acetonitrile in 0.1% (v/v) trifluoroacetic acid results in the baseline separation of A and B chains.

### 2. Results

### 2.1 Proliferative response of peripheral blood T cells to myelin proteins

The short period of only two weeks for expansion of peripheral blood T cells in response to antigen was chosen to minimize selective pressure upon T cells growing in vitro as a result of the experimental protocol or the type of culture medium used. We have found two weeks the minimimum time required to expand sufficient numbers of cells for a significant proliferative response to antigen of the bulk population. In this way, the specificity of the response measured is kept as representative as possible for the full repertoire of T cells responsive to myelin proteins.

The results reveal that by using bulk T cells from peripheral blood as responder cells, significant variations between individual donors may occur. Ten out of twenty-seven samples tested yielded no useful data. Either no growth at all was measured or background proliferation was excessive. Seventeen out of twenty-seven samples from different HLA-typed donors, however, gave useful results in revealing significant proliferation to some HPLC fractions as well as to the complete set of myelin proteins that were used as stimulatory antigen. Three out of five PBMC samples from MS samples patients displayed significant proliferation against myelin proteins. A representative set of data, illustrating responses obtained with five samples, is given in Fig. 2. In the left panel, responses are shown to HPLC-fractionated proteins isolated from control myelin. In the right panel, responses against proteins derived from MS-affected myelin are shown.

The qualitative aspects of the response profiles obtained against control myelin proteins were remarkably similar among the different donors, irrespective of HLA typing or whether or not the donor suffered from definite MS. In all cases, responses were predominant to proteins that elute along the gradient at positions intermediate between the highly hydrophilic MBP (fractions 5 and 6) and the very hydrophobic PLP's (fractions 22-28). Responses to the major proteins MBP and PLP were modest, if at all significant. These findings are in line with our earlier observations [13]. It remains to be established whether or not the apparently predominant responses to the 'intermediate' fractions 10-15 point to the presence of a single immunogenic component or whether they reflect accumulated responses to a multitude of different minor proteins present in these fractions.

Proliferative responses to the set of proteins isolated from MS-affected myelin are similar to those against control material in revealing significant responses to the 'intermediate' fractions 10-15 that contain many different minor myelin proteins. Yet, the highest proliferative response in all samples examined was consistently directed at a single HPLC fraction, viz. fraction 8. Also when supplied at five- or ten-fold dilution did the contents of fraction 8 trigger the highest proliferative response (data not shown). The responses to fraction 8 were dependent on priming with myelin since unprimed PBMC or PBMC primed with an irrelevant antigen (Influenza virus) showed no response. Responses to fraction 8, however, did not depend on priming with MS-affected material since PBMC primed with control myelin showed comparable responses.

It should be noted that no clear differences have been observed thusfar in the overall specificity of peripheral blood T cells from either healthy controls or MS patients. However, the analysis of antigen specificity as performed here provides no data as to precursor frequencies, pre-activation state, fine-specificity or receptor features of T cells in peripheral blood nor data on T cells elsewhere in the body. Thus, myelin-directed T cell responses in MS patients may still be different from control subjects in a number of aspects that have not been addressed in the present study.

Apparently, MS-affected myelin contains one or more highly immunogenic minor myelin antigen(s) for peripheral blood T cells from donors of different HLA type including healthy as well as MS-affected individuals that is/are contained in HPLC fraction 8. Increased levels of such (an) immunogenic protein(s) in this fraction relative to the material obtained from control myelin most likely explains the striking difference observed between the two series of assays illustrated in Fig. 1. Below, the identification of this immunogenic protein is described.

### 2.2 The immunogenic protein in MS-affected myelin is alpha B crystallin

SDS-PAGE analysis as well as additional reversed-phase HPLC analysis of the contents of HPLC fraction 8 containing MS-afected myelin protein revealed the almost exclusive presence of a protein with an apparent mass of 23 kDa. Using a novel preparation of MS-affected myelin proteins as a source, this 23 kDa protein together with other proteins co-eluting at approximately the same position in the HPLC gradient were subfractionated by additional RP-HPLC steps. Again proliferative responses of peripheral blood T cells primed with whole myelin proteins were directed at those HPLC fractions that contained the 23 kDa protein.

The 23 kDa protein was purified to homogeneity by reversed-phase HPLC and subjected to direct amino acid sequencing. No clear signals, however, were recorded upon sequencing suggesting the presence of an N-terminal modification of the protein obstructive to sequencing. In order to allow sequencing of internal protein segments, tryptic fragments were generated from the 23 kDa protein. Six peptides were purified to homogeneity and subjected to sequencing. This resulted in the identification of three separate sequences; two pairs of peptides turned out to share their N-terminal sequence. All three sequences were identical to internal sequences of human alpha B crystallin (Table 1).

Immunoblotting using polyclonal rabbit antibodies raised against the 23 kDa protein purified from human MS-affected brain confirmed the identification of the protein as alpha B crystallin. Western blotting of the SDS-PAGE separated contents of HPLC fraction 8 from MS-affected myelin led to distinct staining only of the predominant 23 kDa protein band in this fraction. This protein band can also be detected in the corresponding HPLC fraction containing proteins from control myelin. Also the alpha crystallin B chain as isolated from a commercial preparation of alpha crystallin from bovine eye lens was recognized upon western blotting by the anti-23 kDa antibodies whereas no recognition could be detected of the A chain of alpha crystallin nor of purified MBP from human MS-affected myelin. Co-migration upon SDS-PAGE was observed between alpha B crystallin from bovine eye lens and the purified 23 kDa human myelin protein from MS-affected myelin. Also upon reversed phase HPLC, the purified 23 kDa protein and alpha B crystallin behaved identically.

**Table 1.**

| **Tryptic fragments of purified human 23 kDa protein contain alpha B crystallin sequences.** | | | | | | | | | | | | | |
|---|---|---|---|---|---|---|---|---|---|---|---|---|---|
| fragment 1 | I | P | A | D | V | D | P | (L) | ... | | | | |
| _{α}B crystallin | I₁₂₄ | P | A | D | V | D | P | L | A | | | | |
| fragment 2: | Y | L | R | (P) | | | | | | | | | |
| _{α}B crystallin | Y₄₈ | L | R | P | | | | | | | | | |
| fragment 3: | A | P | S | W | F | D | T | G | L | S | E | M | R |
| _{α}B crystallin | A₅₈ | P | S | W | F | D | T | G | L | S | E | M | R |

### REFERENCES

[1] Bjorkman PJM, Saper B, Samraoui B, Bennett WS, Strominger JL and Wiley DC (1987) Structure of the human class I histocompatibility antigen. Nature **329**, 506.
[2] Weiss A (1991) Molecular and genetic insights into T-cell antigen receptor structure and function. Ann. Rev. Genet. **25**, 487.
[3] Janeway CA (1992) The immune system evolved to discriminate infectious nonself from noninfectious self. Immunol. Today **13**, 11.
[4] Sinha AA, Lopez MT and McDevitt HO (1990) Autoimmune disease: the failure of selftolerance. Science **248**, 1380.
[5] Hafler DA and Weiner HL (1989) MS: a CNS and systemic autoimmune disease. Immunol. Today **10**, 104.
[6] Raine CS (1991) Multiple sclerosis: a pivotal role for the T cell in lesion development. Neuropathol. Appl. Neurobiol. **17**, 265.
[7] Lees M and Brostoff S (1984) Proteins in myelin. In: Morell P (ed) Myelin, Plenum, New York, pp 197-225.
[8] Van Noort JM, Van Sechel AC, Boon, J, Boersma WJA, Polman CH and Lucas CJ (1993) Minor myelin proteins can be major targets for peripheral blood T cells from both multiple sclerosis patients and healthy subjects. J. Neuroimmunol. **46**, 67.
[9] Campagnoni AT (1988) Molecular biology of myelin proteins from the central nervous system. J. Neurochem. **51**, 1.
[10] Weiner HL and Hafler DA (1988) Immunotherapy of multiple sclerosis. Ann. Neurol.**3**, 211.
[11] Wraith DC, Smilek DE, Mitchell DJH, Steinman L and McDevitt HO (1989) Antigen recognition in autoimmune encephalomyelitis and the potential for peptide-mediated immunotherapy. Cell **59**, 247.
[12] Vandenbark AA, Hashim G, and Offner H (1989) Immunization with a synthetic T-cell receptor V-region peptide protects against experimental allergic encephalomyelitis. Nature **341**, 541.
[13] Van Noort JM, El Ouagmiri M, Boon, J and Van Sechel AC (1994) Fractionation of central nervous system myelin proteins by reversed-phase high-performance liquid chromatography. J. Chromat. **653**, 155.
[14] Iwaki T, Kume-Iwaki A, Goldman JE (1990) Cellular distribution of _{α} B-crystallin in non-lenticular tissue. J. Histochem. Cytochem. **38**, 31.
[15] Ingolia TD and Craig EA (1982) Four small Drosophila heat shock proteins are related to each other and to mammalian _{α}-crystallin. Proc. Natl. Acad. Sci. USA **79**, 2360.
[16] Nicholl ID and Quinlan RA (1994) Chaperone activity of _{α} -crystallins modulates intermediate filament assembly. EMBO j. **13**, 945.
[17] Iwaki T, Iwaki A, Tateishi J, Sakaki Y and Goldman JE (1993) _{α}B crystallin and 27-kd heat shock protein are regulated by stress conditions in the central nervous system and accumulate in Rosenthal fibers. Am. J. Pathol. **143**, 487.
[18] Iwaki T, Wisniewski T, Iwaki A, Corbin E, Tomokane N, Tateishi J and Goldman JE (1992) Accumulation of _{α}B-crystallin in central nervous system glia and neurons in pathological conditions. Am. J. Pathol. **140**, 345.
[19] Bernard CCA and Kerlero de Rosbo N (1992) Multiple sclerosis: an autoimmune disease of multifactorial etiology. Cuur. Opin. Immunol. **4**, 760.
[20] Fierz W, Endler B, Reske K., Wekerle H. and Fontana A. (1985) Astrocytes as antigen presenting cells. I. Induction of Ia antigen expression on astrocytes by T cells via immune interferon and its effect on antigen presentation. J. Immunol. **134**, 3785.
[21] Lanzavecchia A (1993) Strategies to specific immune intervention. Science **262**, 45.
[22] Schwartz RH (1990) A cell culture model for T lymphocyte clonal anergy. Science **248**,1349.
[23] Linsley PS, Wallace PM, Johnson J, Gibson MG, Greene JL, Ledbetter JA, Singh C and Tepper MA (1992) Immunosuppression in vivo with a soluble form of the CTLA-4 T cell activation molecule. Science **257**, 792.
[24] Norton WT and Poduslo SE (1973) Myelination in rat brain: method of myelin isolation. J. Neurochem. **21**, 749.

## Claims

1. Use of alpha B crystallin, or an immunogenic part or derivative thereof, or an antibody to alpha B crystallin, in the diagnosis of auto-immune diseases, wherein said diagnosis comprises determining the presence of alpha B crystallin, or a part of alpha B crystallin, or an antibody to alpha B crystallin, in a body fluid.

2. Use according to claim 1, wherein the auto-immune disease is multiple sclerosis.

3. Alpha B crystallin or at least a specific immunogenic part or derivative thereof for use as a therapeutic agent.

4. Alpha B crystallin or at least a specific immunogenic part or derivative thereof for use in the treatment of auto-immune diseases.

5. Alpha B crystallin or at least a specific immunogenic part or derivative thereof for use in the treatment of Multiple Sclerosis.

6. Alpha B crystallin or at least a specific immunogenic part or derivative thereof for a use according to any one of the claims 3-5, wherein it is used to induce tolerance against alpha B crystallin.

7. Use of alpha B crystallin in the preparation of a medicament for the treatment of auto-immune diseases.

8. Use of alpha B crystallin in the preparation of a medicament for the treatment of Multiple Sclerosis.

9. Antagonist of alpha B crystallin, not being an antibody to alpha B crystallin, wherein said antagonist blocks the immune response to alpha B crystallin.

10. Antagonist according to claim 9, which blocks the interaction between alpha B crystallin and T cells.

11. Antagonist according to claim 10, wherein the T cell is a T helper cell.

12. Auto-reactive T cell specific for alpha B crystallin for use as a therapeutic agent.

13. Auto-reactive T cell specific for alpha B crystallin for use in the treatment of Multiple Sclerosis.

14. Auto-reactive T cell specific for alpha B crystallin according to claim 12 or 13, which has been inactivated.

15. Use of an auto-reactive T cell to alpha B crystallin in the preparation of a medicament for the treatment of Multiple Sclerosis.

16. A vaccine for use in the treatment of auto-immune diseases comprising an inactivated auto-reactive T cell directed to alpha B crystallin.

17. T cell receptor of a part or a derivative thereof obtainable from a T cell according to claim 12-14, for use in the treatment of an auto-immune disease, in particular Multiple Sclerosis.

18. A vaccin for use in the treatment of an auto-immune disease, in particular Multiple Sclerosis comprising a T cell receptor or a part or a derivative thereof according to claim 17.

## Patentansprüche

1. Verwendung von alpha B Crystallin oder eines immunogenen Teils oder Derivats desselben oder eines Antikörpers gegen alpha B Crystallin zur Diagnose von Auto-Immunerkrankungen, wobei die Diagnose den Nachweis von alpha B Crystallin oder eines Teils von alpha B Crystallin oder eines Antikörpers gegen alpha B Crystallin in einer Körperflüssigkeit umfaßt.

2. Verwendung nach Anspruch 1, wobei die Auto-Immunerkrankung Multiple Sklerose ist.

3. Alpha B Crystallin oder wenigstens ein spezifischer immunogener Teil oder ein Derivat desselben zur Verwendung als therapeutisches Agens.

4. Alpha B Crystallin oder wenigstens ein spezifischer immunogener Teil oder ein Derivat desselben zur Verwendung bei der Behandlung von Auto-Immunerkrankungen.

5. Alpha B Crystallin oder wenigstens ein spezifischer immunogener Teil oder ein Derivat desselben zur Verwendung bei der Behandlung von multipler Sklerose.

6. Alpha B Crystallin oder wenigstens ein spezifischer immunogener Teil oder ein Derivat desselben zur Verwendung nach einem der Ansprüche 3 bis 5, wobei es verwendet wird, um gegenüber alpha B Crystallin Toleranz zu induzieren.

7. Verwendung von alpha B Crystallin zur Herstellung eines Medikaments zur Behandlung von Auto-Immunerkrankungen.

8. Verwendung von alpha B Crystallin zur Herstellung eines Medikaments zur Behandlung von multipler Sklerose.

9. Antagonist von alpha B Crystallin, der kein Antikörper gegen alpha B Crystallin ist, wobei der Antagonist die Immun-Antwort gegen alpha B Crystallin blockiert.

10. Antagonist nach Anspruch 9, der die Wechselwirkung zwischen alpha B Crystallin und T-Zellen blockiert.

11. Antagonist nach Anspruch 10, wobei die T-Zelle eine T-Helfer-Zelle ist.

12. Auto-reaktive T-Zelle, die für alpha B Crystallin spezifisch ist, zur Verwendung als therapeutisches Agens.

13. Auto-reaktive T-Zelle, die für alpha B Crystallin spezifisch ist, zur Verwendung bei der Behandlung von Multipler Sklerose.

14. Auto-reaktive T-Zelle nach Anspruch 12 oder 13, die für alpha B Crystallin spezifisch ist und die inaktiviert worden ist.

15. Verwendung einer auto-reaktiven T-Zelle gegen alpha B Crystallin zur Herstellung eines Medikaments zur Behandlung von Multipler Sklerose.

16. Vakzine zur Verwendung bei der Behandlung von Auto-Immunerkrankungen, die eine gegen alpha B Crystallin gerichtete inaktivierte auto-reaktive T-Zelle umfaßt.

17. T-Zell-Rezeptor oder ein Teil oder ein Derivat desselben, erhältlich von einer T-Zelle nach den Ansprüchen 12 bis 14 zur Verwendung bei der Behandlung einer Auto-Immunerkrankung, insbesondere von Multipler Sklerose.

18. Vakzine zur Verwendung bei der Behandlung einer Auto-Immunerkrankung, insbesondere von Multipler Sklerose, welche einen T-Zell-Rezeptor oder einen Teil oder ein Derivat desselben nach Anspruch 17 umfaßt.

## Revendications

1. Utilisation de cristalline alpha B, d'une partie immunogène de cristalline alpha B, d'un dérivé ou d'un anticorps de cristalline alpha B, dans le diagnostic de maladies auto-immunes, caractérisée en ce que ledit diagnostic comporte la détermination de la présence de cristalline alpha B, d'une partie de cristalline alpha B ou d'un anticorps de la cristalline alpha B, dans un fluide corporel.

2. Utilisation selon la revendication 1, caractérisée en ce que la maladie auto-immune est la sclérose en plaques.

3. Cristalline alpha B ou au moins une partie immunogène spécifique ou un dérivé de celle-ci destinée à être utilisée comme agent thérapeutique.

4. Cristalline alpha B ou au moins une partie immunogène spécifique ou un dérivé de celle-ci destinée à être utilisée dans le traitement de maladies auto-immunes.

5. Cristalline alpha B ou au moins une partie immunogène spécifique ou un dérivé de celle-ci destinée à être utilisée dans le traitement de la sclérose en plaques.

6. Utilisation de cristalline alpha B ou au moins une partie immunogène spécifique ou un dérivé de celle-ci selon l'une quelconque des revendications 3 à 5, caractérisée en ce qu'elle induit une tolérance vis-à-vis de la cristalline alpha B.

7. Utilisation de cristalline alpha B dans la préparation d'un médicament pour le traitement de maladies auto-immunes.

8. Utilisation de cristalline alpha B dans la préparation d'un médicament pour le traitement de la sclérose en plaques.

9. Antagoniste de la cristalline alpha B, n'étant pas un anticorps de la cristalline alpha B, caractérisé en ce que ledit antagoniste bloque la réponse immunitaire à la cristalline alpha B.

10. Antagoniste selon la revendication 9, qui bloque l'interaction entre la cristalline alpha B et les cellules T.

11. Antagoniste selon la revendication 10, caractérisé en ce que la cellule T est une cellule T auxiliaire.

12. Cellule T auto-réactive spécifique de la cristalline alpha B destinée à être utilisée comme agent thérapeutique.

13. Cellule T auto-réactive spécifique de la cristalline alpha B destinée à être utilisée dans le traitement de la sclérose en plaques.

14. Cellule T auto-réactive spécifique de la cristalline alpha B selon la revendication 12 ou 13, qui a été inactivée.

15. Utilisation d'une cellule T auto-réactive à la cristalline alpha B dans la préparation d'un médicament pour le traitement de la sclérose en plaques.

16. Vaccin destiné à être utilisé dans le traitement de maladies auto-immunes comprenant une cellule T auto-réactive inactivée dirigée vers la cristalline alpha B.

17. Récepteur de cellule T ou une partie ou un dérivé de celui-ci que l'on peut obtenir à partir d'une cellule T selon les revendications 12 à 14, destinée à être utilisée dans le traitement d'une maladie auto-immune, notamment la sclérose en plaques.

18. Vaccin destiné à être utilisé dans le traitement d'une maladie auto-immune, notamment la sclérose en plaques, comprenant un récepteur de cellule T, une partie ou un dérivé de celui-ci selon la revendication 17.
